# EUROPEAN PATENT SPECIFICATION

(11) **EP 3 480 302 B1**
(45) Date of publication and mention of the grant of the patent: **24.11.2021**
(21) Application number: 16907053.9
(22) Date of filing: 12.10.2016
(51) Int. Cl.: C12N 5/10, C12N 15/85, G01N 33/68, C12R 1/91

(54) **METHOD FOR CONSTRUCTING CELL MODEL FOR DETECTING PYROGEN, CELL MODEL AND PYROGEN DETECTION KIT**
VERFAHREN ZUR HERSTELLUNG EINES ZELLMODELLS FÜR DEN NACHWEIS VON PYROGEN, ZELLMODELL UND PYROGENNACHWEISKIT
PROCÉDÉ DE CONSTRUCTION D'UN MODÈLE CELLULAIRE POUR LA DÉTECTION DE PYROGÈNE, MODÈLE CELLULAIRE ET KIT DE DÉTECTION DE PYROGÈNE

(30) Priority: 29.06.2016 CN 201610496477
(43) Date of publication of application: 08.05.2019
(73) Proprietor: Niu, Gang, Beijing 100082 (CN)
(72) Inventor: TAN, Huanran, Beijing 100082 (CN); NIU, Gang, Beijing 100082 (CN)
(74) Representative: Cabinet Beau de Loménie
(86) International application number: PCT/CN2016/101862
(87) International publication number: WO 2018/000657

(56) References cited:
- WO-A1-2012/128580
- WO-A2-2005/089122
- CN-A- 102 796 706
- CN-A- 105 567 735
- US-A1- 2009 253 134
- LIZUNDIA REOINA ET AL: "Host species-specific usage of the TLR4-LPS receptor complex", INNATE IMMUNITY, SAGE PUBLICATIONS LTD, US, vol. 14, no. 4, 1 August 2008 (2008-08-01) , pages 223-231, XP009128098, ISSN: 1753-4259, DOI: 10.1177/1753425908095957
- FABIAN OCEGUERA-YANEZ ET AL: "Engineering the AAVS1 locus for consistent and scalable transgene expression in human iPSCs and their differentiated derivatives", METHODS, vol. 101, 18 December 2015 (2015-12-18), pages 43-55, XP055456602, NL ISSN: 1046-2023, DOI: 10.1016/j.ymeth.2015.12.012
- KENNEDY M N ET AL: "A complex of soluble MD-2 and lipopolysaccharide serves as an activating ligand for Toll-like receptor 4", JOURNAL OF BIOLOGICAL CHEMISTRY,, vol. 279, no. 33, 13 August 2004 (2004-08-13), pages 34698-34704, XP002323011, ISSN: 0021-9258, DOI: 10.1074/JBC.M405444200
- LEI, ZHEN: "Pyrogen Test Platform of Drugs-the Construction of Stable Expression TLR4, MD 2and CD 14Genes Cell Line", Thesis for Application of Master s Degree,, 15 March 2015 (2015-03-15), pages 1-96, XP009513254, CN ISSN: 1674-0246
- GIUSEPPE MANCUSO et al.: "Bacteroides fragilis-Derived Lipopolysaccharide Produces Cell Activation and Lethal Toxicity via Toll-Like Receptor 4", INFECTION AND IMMUNITY, vol. 73, no. 9, 30 September 2005 (2005-09-30), pages 5620-5627, XP055459828, ISSN: 0019-9567
- QIAN, LEIYANG et al.: "Research Status of Novel Gene Editing Technology of CRISPR/Cas9 System", Journal of Hebei University ( Natural Science Edition, vol. 36, no. 1, 28 February 2016 (2016-02-28), pages 84-93, ISSN: 1000-1565

### SEQUENCE LISTING

<110> Niu Gang
<120> Construction method of cell models for detecting pyrogens, cell
   models and pyrogen detection kits
<160> 30
<170> PatentIn version 3.3
<210> 1
   <211> 26
   <212> DNA
   <213> Artificial sequence
<400> 1
   gcgaattcca tgttaccatt tctgtt 26
<210> 2
   <211> 27
   <212> DNA
   <213> Artificial sequence
<400> 2
   ccggtacctt gcaatttgaa ttaggtt 27
<210> 3
   <211> 29
   <212> DNA
   <213> Artificial sequence
<400> 3
   tagaattcat ggagcgcgcg tcctgcttg 29
<210> 4
   <211> 27
   <212> DNA
   <213> Artificial sequence
<400> 4
   ttggtacctc gaaggcaaag ccccggg 27
<210> 5
   <211> 27
   <212> DNA
   <213> Artificial sequence
<400> 5
   tcggtaccga gctcggatcc atgatgt 27
<210> 6
   <211> 28
   <212> DNA
   <213> Artificial sequence
<400> 6
   atcccgggtg ctcaccatct cgaggata 28
<210> 7
   <211> 34
   <212> DNA
   <213> Artificial sequence
<400> 7
   gatcgggggc cacuagggac aggaugtttt agag 34
<210> 8
   <211> 34
   <212> DNA
   <213> Artificial sequence
<400> 8
   ctagctctaa aacauccugu cccuaguggc cccc 34
<210> 9
   <211> 23
   <212> DNA
   <213> Artificial sequence
<400> 9
   acttttatct gtcccctcca ccc 23
<210> 10
   <211> 23
   <212> DNA
   <213> Artificial sequence
<400> 10
   catccctgtg aaagatgcct gag 23
<210> 11
   <211> 23
   <212> DNA
   <213> Artificial sequence
<400> 11
   tcttccgcat tggagtcgct tta 23
<210> 12
   <211> 27
   <212> DNA
   <213> Artificial sequence
<400> 12
   gtaagcttga ggggacagat aaaagta 27
<210> 13
   <211> 54
   <212> DNA
   <213> Artificial sequence
<400> 13
   cagctgttga attttgacct tcttaagctt gcgggagacg tcgagtccaa ccct 54
<210> 14
   <211> 54
   <212> DNA
   <213> Artificial sequence
<400> 14
   gagggcagag gaagtcttct aacatgcggt gacgtggagg agaatcccgg ccct 54
<210> 15
   <211> 87
   <212> DNA
   <213> Artificial sequence
<400> 15
   cgggcccggg atccacagct gttgaatttt gaccttctta agcttgcggg agacgtcgag 60
   tccaacccta tggcctcctc cgaggac 87
<210> 16
   <211> 35
   <212> DNA
   <213> Artificial sequence
<400> 16
   ctgaggagtg cggccctaca ggaacaggtg gtggc 35
<210> 17
   <211> 18
   <212> DNA
   <213> Artificial sequence
<400> 17
   tttggcaaag aattcatg 18
<210> 18
   <211> 53
   <212> DNA
   <213> Artificial sequence
<400> 18
   acgtcaccgc atgttagaag acttcctctg ccctcctgat ttgaattagg ttg 53
<210> 19
   <211> 34
   <212> DNA
   <213> Artificial sequence
<400> 19
   gatcgugagg gcuuaacaca gugccgtttt agag 34
<210> 20
   <211> 34
   <212> DNA
   <213> Artificial sequence
<400> 20
   ctagctctaa aacggcacug uguuaagccc ucac 34
<210> 21
   <211> 19
   <212> DNA
   <213> Artificial sequence
<400> 21
   tggtaacagt tagccactt 19
<210> 22
   <211> 19
   <212> DNA
   <213> Artificial sequence
<400> 22
   gattcttcat cttcccttt 19
<210> 23
   <211> 21
   <212> DNA
   <213> Artificial sequence
<400> 23
   aacacctcct actatttact g 21
<210> 24
   <211> 22
   <212> DNA
   <213> Artificial sequence
<400> 24
   agccctcaac atgcaatttc tc 22
<210> 25
   <211> 17
   <212> DNA
   <213> Artificial sequence
<400> 25
   tcttcttcct ccaaccc 17
<210> 26
   <211> 18
   <212> DNA
   <213> Artificial sequence
<400> 26
   gacccaatat caggagac 18
<210> 27
   <211> 19
   <212> DNA
   <213> Artificial sequence
<400> 27
   ggctatgaac taatgaccc 19
<210> 28
   <211> 17
   <212> DNA
   <213> Artificial sequence
<400> 28
   cgggaatgct ttgtatc 17
<210> 29
   <211> 16
   <212> DNA
   <213> Artificial sequence
<400> 29
   tgcccgctat gtctaa 16
<210> 30
   <211> 20
   <212> DNA
   <213> Artificial sequence
<400> 30
   atgggctatg aactaatgac 20

## Description

### Technical Field

The present invention belongs to the biotechnology field, and relates to the detection of pyrogens in drugs and biological products (including gene engineering products), in particular to a construction method of a cell model for detecting pyrogens, a cell model and a pyrogen detection kit.

### Background Art

### 1.1 Overview of pyrogens

It is generally known that the use of injection drugs or medical devices in clinical treatment may often cause adverse reactions such as cold, chill, nausea, fever, vomiting, headache, leukopenia, increased vascular permeability, coma and shock, leading to disease progressions, which can cause patient discomfort, render the rescue and treatment ineffective, or even be life-threatening. In 1875, the study by Burdon-Sanderson (Li Zenghong, Wang Ruidong, Journal of Foshan University, 2007, 25(3):43-45) showed that a substance free of living bacteria (isolated from rotten meat) can cause a series of adverse reactions mentioned-above, which was called as pyrogen, and these adverse reactions were defined as pyrogenic reactions. Then, with the further development of scientific researches, pyrogen had a more precise definition: a substance, produced by microorganisms, that causes a rise in temperature of a warm-blooded animal body. In 1923, Seibert affirmed that pyrogen was produced by microorganisms and featured by heat resistance, non-volatility, water solubility, filterability and adorability, was hard to be retained, and showed strong heat stability, and in the case of strict sterilization, the occurrence of pyrogenic reactions can not yet be avoided. Pyrogen is classified into two types: exogenous pyrogen and endogenous pyrogen, exogenous pyrogen is considered to be endotoxin of Gram-negative bacteria and lipoteichoic acid of Gram-negative bacteria, and endogenous pyrogen is considered to be cytokines (such as TNF-α, INF-β, IL-1, growth factors), hormones (such as steroids and prostaglandins) in an organism. While, what we usually call 'pyrogens' mainly refers to bacterial pyrogens that are metabolites of some bacteria, dead bacteria and endotoxins. Among them, the metabolites of Gram-negative G-bacilli have the strongest pyrogenicity, followed by Gram-positive G+bacilli, Gram-positive G+cocci, fungi, yeast and even viruses may produce pyrogens (Charles A, Dinarello. Endotoxin Research, 2004, 10:201).

Endotoxin, as exogenous pyrogen, is an unique structure on the outer wall of Gram-negative G-bacilli, being a composition of phospholipids, lipopolysaceharide (LPS) and proteins. LPS, as the key component and active center of the composition, has the strongest pyrogenicity, due to the difference in chemical constitution among different types of bacteria,, the molecular weight of LPS ranges from 5 × 10⁴ to 5 × 10⁵, and the greater the molecular weight, the stronger the pyrogenicity. Endotoxin, instead of bacteria themselves or metabolites of bacteria, is a type of biologically active toxic substances resulting from the death or destruction of bacteria, which can not be eliminated by the common sterilization methods, and is one of the most widely bioactive substances ever found so far (Liu Xuanya, Journal of Gelatin Science and Technology, 2013,33(1):45-48). When entering a human body through the digestive system, endotoxin does not produce harm to the human body, but if entering the blood of a human body by injection, endotoxin may cause various diseases, the most typically pyrogenic reactions. Endotoxin that enters a human body can not only directly cause toxicity to cell membranes but also induce the synthesis and release of a variety of cytokines and inflammatory factors through immune inflammatory response mediated by mononuclear macrophages, and these inflammatory mediators that are overexpressed act on receptors on the monocyte-macrophage membranes by autocrine, paracrine or endocrine action, further activating the expression of inflammatory mediators (Guha M, Mackman N. Cell Signal, 2001, 13(2):85-94.). The uncontrolled release of these inflammatory factors affects the integrity of cell functions, causing metabolic disorders of an organism, increased blood coagulation and inadequate tissue perfusion, even causing death due to multiple organ failure (Hotchkiss RS, Karl IE. N Engl J Med, 2003, 348(2):138-150.). With the increase of attention to clinical pyrogenic reactions in drug and medical management departments, as the distribution of Gram-negative bacteria is very extensive, the detection of pyrogens in drugs and medical devices has become the focus of recent researches.

### 1.2 Main pyrogen detection methods in the prior art and limitations thereof

For the detection of pyrogens, the methods available in the Chinese Pharmacopoeia include rabbit pyrogen test method and Limulus amebocyte lysate endotoxin test method, and in addition, a new method, monocyte-activation test, is recorded in the European Pharmacopoeia (7th edition, 2011). Rabbit pyrogen test method As rabbits have a substantially similar response to pyrogens as human beings, the rabbit pyrogen test method is a legal recommended one for pyrogen detection specified in the pharmacopoeias of various countries. A certain amount of a test sample is intravenously injected into a rabbit body, the condition of body temperature rise of the rabbit is observed within a set period of time, to determine whether the limit of pyrogens contained in the test sample complies with the requirements specified.

However, there are still many limitations in the rabbit pyrogen test method. Firstly, the rabbit pyrogen test method can not be quantified or standardized and has poor sensitivity and repeatability, and this method also has inter-individual difference in sensibility to pyrogens, with individual difference in sensibility to pyrogens up to more than 10 times. Also, as some types of drugs may influence the detection results, the rabbit pyrogen test method can not be applied to, for example, some antipyretic analgesic drugs, cytotoxic drugs or some drugs that affects the body temperature center. Further, many products that have passed the detection by the rabbit pyrogen test method may still cause pyrogenic reactions. Today, with the rapid development and fast change of cell engineering technology, new biological materials and traditional Chinese medicine products, the rabbit pyrogen test method can not fully meet the needs for fever reactions in a human body, is time-consuming and complicated, is unsuitable for the quality control in the production of injection products, and is not applied to some drugs.

Limulus amebocyte lysate endotoxin test method When Limulus amebocyte lysate (extract of blood cells from Limulus polyphemus) is exposed to Gram-negative bacteria, coagulation is caused, therefore, the Limulus amebocyte lysate endotoxin test method is currently recognized by major pharmacopoeias in America, Britain, Europe, Japan and China. Limulus amebocyte lysate endotoxin test method is used to detect or quantify endotoxins generated by Gram-negative bacteria using Limulus amebocyte lysate, so as to determine whether the limit of endotoxins in a test sample complies with the requirements specified. The amount of endotoxins is represented by endotoxin unit (EU). The Limulus amebocyte lysate endotoxin test method is superior in sensitivity of endotoxin detection to the rabbit pyrogen test method, being 0.015EU/mL, and easy and feasible to operate, has low test cost, provides outcomes rapidly and reliably, is suitable for pyrogen control in the production of injection products, and can be applied to some cytotoxic drug products that can not be detected by the rabbit pyrogen test method.

But the Limulus amebocyte lysate endotoxin test method is seriously influenced by environmental factors, resulting in false positive results. And it can not be applied to pharmaceutical products having colors. Actually, the Limulus amebocyte lysate endotoxin test method is able to detect the coagulation of Limulus polyphemus blood exposed to endotoxins rather than the pyrogenic activity or content at chemical level of endotoxins in a human body, and fails to detect other pyrogens than endotoxins. This method also fails to detect some types of enzyme inhibitors and Ca-Mg ion chelating agents, therefore, the Limulus amebocyte lysate endotoxin test method can not completely replace the rabbit pyrogen test method. Tachypleus tridentatus that is used in the Limulus amebocyte lysate endotoxin test method in our country is a kind of species appearing prior to dinosaurs, but currently threatened with extinction due to land reclamation and fishery activities. Based on '3Rs Rule' in experimental zoology (namely Reduction, Refinement and Replacement), the study on substitutes for Limulus amebocyte lysate is extremely urgent. Monocyte activation test method In addition to the rabbit pyrogen test method and the Limulus amebocyte lysate endotoxin test, a new method, monocyte activation test (MAT) has been recorded in the European Pharmacopoeia (7th edition, 2011). The principle is that: the whole blood of a human being is incubated with a test sample, and then the activity of pyrogens in the test sample is evaluated based on the amounts of IL-6, IL-1β, TNF-α and other cell factors released from the monocytes in the blood. The monocyte activation test (MAT) can detect pyrogens and proinflammatory cytokines (including endotoxins from Gram-negative bacteria) and non-endotoxin derived foreign matters (including biological or chemical entities associated with pathogen associated molecular patterns (PAMPs) and products of Gram-positive bacteria, Gram-negative bacteria, viruses and fungi and biological or chemical entities associated with processing technologies). However, the monocyte activation test (MAT) also has some limitations, for example, monocytes from different human blood sources may be different in reactivity, and fresh human blood is difficult to get in batch. Accordingly, this method is hard to be popularized, and it also has poor stability.

Thus it can be seen that the use of a new in vitro pyrogen detection method based on gene engineering instead of the conventional method is an international trend of development in this field. The study on pyrogen detection develops at the cell and molecular level. The study on pyrogen detection in our country is still in the primary stage, and cell models for pyrogen detection have not yet recorded in Chinese Pharmacopoeia. However, it can be predicted that cell models for pyrogen detection have become the concern focus of more and more researchers, Considering feasible operation and high efficiency is the inevitable trend of this study.

### 1.3 Pyrogen-associated receptors and signaling pathways

In recent years, the mechanism of response to endotoxins from Gram-negative bacteria (G-bacteria) has been deeply understood, and LPS that activates an immune response in an organism and plays a leading role in inducing pyrogenic reaction has been well recognized. LPS presents some highly-conserved pathogen structures in various pathogens, and these conserved pathogen structures are called 'pathogen associated molecular patterns' (PAMP). Toll-like receptors, as an important component of congenital immune system, are considered to be similar to pattern-recognition receptors (PRRs), initiating an immune response by identifying PAMP on the surface of a pathogenic organism, to eliminate a foreign antigen.

In an LPS-induced signaling pathway of inflammation, the following four receptors play a key role: lipopolysaccharide binding protein (LBP), Toll like receptor-4 (TLR4), myeloid differentiation protein-2 (MD2) and monocyte differentiation antigen 14 (CD14).

LBP is a kind of glycoproteins which wildly exist in human and animal blood sera, but technically, LPS binding proteins are not proteins binding with LPS, however, LPS-mediated cell activation requires proteins capable of binding with LPS, which are important carrier for LPS playing a biological role. It has high affinity to endotoxins and lipid A in LPS. LBP and LPS combine to form a complex which in turn delivers LPS to the CD14 receptor on a cell membrane, causing a series of transmembrane signal transduction process and intracellular signal transduction process in combination with the TLR4-MD2 complex, as a result, target cells are activated and proinflammatory cytokines and immunomodulatory cytokines are released.

TLR4 is a member of Toll like receptors, so far, 10 types of proteins of TLRs have been found and named as TLR1-10 separately (Lemaitre B, Nicolas E, Miehaut L et al. Cell, 1996,86(6):973-983; HuangB. Zhao J. Unkeless J C et al. Oneogene, 2008,27(2):218-224; Liu Xing, Feng Wenli, Kang Gefei, Foreign Journal of Clinical Biochemistry and Laboratory Science, 2001, 22(3):134-136; T Kawai, S Akira. Cell Death and Differentiation, 2006, 13:816-825). Different TLRs transduction signals are stimulated by microorganisms from different sources. Thus, different types of TLRs can identify and distinguish, to a certain extent, the types of pathogens invading an organism. Among TLRs, TLR4 and TLR2 are most frequently studied to determine the role in congenital immunity of an organism, and being an important role in LPS signal transduction, TLR4 is studied widely and deeply (Qin Yuxin, Zhang Qingzhu. Chinese Pharmacological Bulletin, 2003, 19(12):1336-1339; Li-Yun Huang, James L.DuMontelle,et al. Clinlcal Microbiology, 2009, 47:3427-3434). TLR4, as the most important type of pattern-recognition receptors in inflammatory reaction, can specifically identify some conserved sequences (such as LPS) in the evolution process of microorganisms. TLR4, as the most important receptor in LPS-induced cell signal transduction, is mainly expressed on cells involved in host defense functions, such as peripheral blood leukocytes, T lymphocytes, B lymphocytes, monocyte macrophages, mast cells, Langerhans cells, dendritic cells and so on, particularly, the expression of TLR4 on myelomonocytes is most (Medzhitov R, Preston Hurlburt P, Janeway Jr. CA. Nature, 1997, 388:394-79).

MD2 is a kind of secretory proteins, and the immunoprecipitation test using transfectants capable of expressing TLR4 and MD2 has shown that MD2 accompanies with the expression of TLR4, is secreted on the surface of cells and anchored to cells membrane by physical action on TLR4. MD2 is helpful for TLR4 in the identification of LPS, locks LPS on the binding site on TLR4, and plays a very important role in immigration to cell membrane and expression of TLR4. Studies have found that, in the case of lacking MD2, the response of TLR4 to LPS is extremely low (Miyake K, R. Shimazu, J.Kondo,et al. Immunol. 1998, 161:1348-1353; Pugin J, Stem Voeffray S, Daubeuf B. Blood. 2004, 104(13):4071-4079; Liu Yawei, Liu Jinghua, Tang Jing, et, al. Journal of Southern Medical University, 2006, 26(8): 1101-1105; Zhong Tianyu, Liu Jinghua, Jiang Yong. Progress in Biochemistry and Biophysics 2007, 34(5): 460-464).

CD14, as a kind of proteins that are expressed as glycosylphosphatidylinositol anchor connexin on kytoplasm and leucocyte surface, has high affinity to LPS, however, CD14 can not directly transmit LPS signals into cells due to the lack of cytoplasmic domain. LPS and CD14 combines to form LPS-LBP-CD14 ligand and helper factor complex which in turn combine with TLR4 to transmit signals into cells (Saitoh S, Akashi S, Yamada T, et al. Endotoxin Res, 2004, 10 (4): 257-260; Nagai Y, Akashi S, Nagafuku M, et al. Nat Immunol, 2002, 3(7): 667-672; Zielger, Heitbrock HWL and Ulevitch RJ. Immunol Today, 1993, 14(3): 121-125).

Following the invasion of bacteria into an organism, LPS is released into the blood by the destruction of bacteria, combines with free lipopolysaccharide binding protein (LBP) in the plasma to form a complex which is in turn combines with CD14 or directly combines with TLR4 auxiliary protein (i.e. myeloid differentiation protein-2, MD-2) to form LPS-MD2/TLR4 complex, as a result, TLR4 is activated under the action of these molecules.

CN 102796706 A (CHINA-AUSTRILIA SHEEP RESEARCH CENTRE, ANIMAL SCIENCE ACADEMY OF XINJI) 28 November201 2 (2012-11-28) discloses a cell model that can be used for e.g. analysis of the LPS reaction, wherein said cell model is a HEK293 cell that is transfected with a vector that comprises the MD2, CD14 and TLR4 genes in one plasmid (pLEX-MD2-CD14-TLR4).

### 1.4 Fundamental principle of CRISPR/CAS9 system

CRISPR/Cas9 (The Clustered Regularly Interspaced Short Palindromic Repeats (CRISPR) and CRISPR Associated (Cas) system is a new method that can target any gene in human genomes in a rapid, convenient and efficient way. CRISPRs, as a kind of repetition structures that are widely distributed in bacterial and archaeal genomes, refer to clustered regularly inter-spaced short palindromic repeats, which are a DNA fragment developed to fight against bacteriophages and viruses, serving as an adaptive immune protection mechanism of bacteria. Along with the expression of Cas9 in bacteria, the transcription of CRISPR RNA is induced, this RNA being able to pair with the genome of viruses. This Cas9 complex can digest the genome of viruses, resulting in the inactivation of viruses. Studies have shown that CRISPR together with a series of associated proteins and leader sequences can endow prokaryotes with acquired immunity against bacteriophages and other foreign genes. The CRISPR-Cas9 technology works by inducing the identification and digestion of Cas9 nuclease to a specifically targeted DNA using a single directed RNA (sgRNA) that is the same as the target sequence, resulting in DNA double-and single-strand breakage, then, the broken DNA is repaired by cells that own two repair mechanisms, namely, non-homologous end joining (NHEJ) or homology-directed repair (HDR).

The development of CRISPR/Cas system provides a new powerful tool for gene engineering, which brings a breakthrough revolution for the research and application of genome directed the CRISPR/Cas system will help people understand the gene functions better.

Endotoxin, as the primary cause of pyrogenic reaction caused by Gram-negative bacteria, is the key factor posing serious threat to drug safety, therefore, the establishment of a more rational and feasible pyrogen detection method appears especially urgent.

### Summary of the Invention

An object of the present invention is to provide a cell model for detecting pyrogens, which can be used for the detection of drugs and biological products, including gene engineering drugs. Another object of the present invention is to provide a construction method of the cell model for detecting pyrogens.

The third object of the present invention is to provide a kit for detecting pyrogens, which contains the cell model mentioned above.

The fourth object of the present invention is to provide a method for detecting pyrogens, which is implemented by using the kit mentioned above.

In order to achieve the purposes, the present invention adopts the technical solutions as follows. The present invention provides a construction method of a cell model for detecting pyrogens, including the following steps of:
1) assembling TLR4 genome into an expression vector capable of expressing a green fluorescent protein, to construct a recombinant plasmid containing TLR4 gene;
2) knocking the TLR4 gene in a directed manner into an intron of a gene on a chromosome of a cell line by the CRISPR/CAS9 method, to obtain a cell line capable of expressing the TLR4 gene stably;
3) segregating CD14, MD2 and red fluorescent RFP genes using two different 2A peptides, to construct a recombinant plasmid containing CD14 and MD2 genes and capable of expressing a red fluorescent protein;
4) knocking the CD14 and MD2 genes in another intron of a gene on another chromosome than the chromosome on which the TLR4 gene is knocked-in in step 2) by the CRISPR/CAS9 method, to obtain a cell line capable of expressing the TLR4, CD14 and MD2 genes stably and capable of expressing green and red fluorescent proteins, namely the cell model.

Preferably, the cell line is cell line HEK293T, HEK293 or NIH3T3.

Further, the position where the TLR4 gene is knocked-in in step 2) is the first intron of PPP1R12C gene of chromosome 19.

Further, the position where the CD14 and MD2 genes are knocked-in in step 4) is the first intron of CCR5 gene of chromosome 3.

The present invention also provides a cell model constructed by the construction method mentioned above, which is obtained by knocking the TLR4 gene in the first intron of PPP1R12C gene of chromosome 19 and knocking the CD14 and MD2 genes in the first intron of CCR5 gene of chromosome 3 of the cell line HEK 293T.

Wherein, the systematic name of the cell model is human embryonic kidney (HEK) cell variant 293T/TLR4/CD14/MD2, preserved in May 19, 2016 in China General Microbiological Culture Collection Center (CGMCC), Institute of Microbiology, Chinese Academy of Sciences, No.1 Beichen West Road, Chaoyang District, Beijing, with the preservation number CGMCC No. 12296.

The present invention also provides a kit for detecting pyrogens, including 1) a cell model constructed by the construction method mentioned above, or the cell model mentioned above, 2) a pyrogen standard, 3) an IL6 and/or TNFα control.

Preferably, the pyrogen standard is LPS freeze-dried powder, the IL6 control is IL6 freeze-dried powder, and the TNFα control is TNFα freeze-dried powder.

The present invention further provides a method for detecting pyrogens, including the following steps of:
1) preparing a test sample solution and a pyrogen standard solution;
2) adding the test sample solution into a culture medium containing a cell model constructed by the construction method mentioned above, or the cell model mentioned above, and culturing at 37°C for 5 h;
3) collecting supernatant of the culture product, and detecting the contents of IL-6 and/or TNF-α in the supernatant.

Preferably, detecting the contents of IL-6 and/or TNF-α in step 3) is implemented by Western Blot, ELISA, gold standard method or mass spectrometry.

In the present invention, the three genes are knocked in the cells by the CRISPR/CAS9 method, among which two genes are knocked in the same one chromosome in a directed manner and the other one is knocked in another chromosome. The knocked-in TLR4 gene is traced by the green fluorescent protein (GFP). The directed knocked-in CD14-MD2 genes are traced by the red fluorescent protein (RFP). The CD14, MD2 and RFP genes are segregated by the self-shearing 2A peptide multi-gene vector construction strategy using two different 2A peptides, thereby avoiding the impact on protein expression.

In the embodiments of the present invention, two 2A peptides, namely F2A and T2A, are used, in which F2A is a 2A peptide derived from foot-and-mouth disease viruses (FMDVs), T2A is a 2A peptide derived from Theiler's murine encephalitis viruses (TMEVs), thereby avoiding the failure in cloning into the target locus due to the cross reaction of gene sequences of the same one 2A peptide.

After the model cell line is exposed to pyrogens, the release of IL-6 and/or TNF-α cytokines can be detected by ELISA, Western Blot, mass spectrometry and magnetic bead method.

The features of the present invention are as follows.

### Directed knock-in of specific genes by the CRISPR/CAS9 method

In the present invention, the introduction of foreign genes into the cells is implemented by the directed knock-in method instead of virus- or lentivirus-medicated transient transfection, thereby ensuring genetic stability. The existing CRISPR/CAS9 method is typically applied to gene knocking-out or gene mutation rather than gene knock-in, has the problems such as needs for overcoming the off-target effect, low efficiency in homologous recombination, complicated operation of knock-in vector construction, particularly big difficulty in knock-in multiple genes. However, the gene knock-in method adopted by the present invention succeeds in the introduction of multiple genes into a cell line (such as cell line HEK293T), and overcomes the difficulty in gene knock-in of the CRISPR/CAS9 method.

### Knocking-in of genes into different chromosomes

In the present invention, the three genes, namely TLR4, CD14 and MD2 genes, are knocked into two different chromosomes, and the knock-in positions are defined to avoid the impact on the physiological functions of cells and ensure the normal survival and stable passage of cells. For example, in the embodiments of the present invention, the TLR4 gene is inserted into the first intron of PPP1R12C gene of chromosome 13 of the cell line HEK 293T, and the CD14 and MD2 genes are inserted into first intron of CCR5 gene of chromosome 3 of the cell line HEK 293T, these two positions being defined to avoid the impact of gene knock-in on the physiological functions of cells and ensure the normal survival and stable passage of cells. Also, these three genes are not inserted into the same one chromosome, which avoids the impact of steric effect on the gene expression in the prior art that different genes are inserted into the same one chromosome, and the fusion protein of the three genes is too large, which may affect the gene expression on the chromosomes. The genes inserted into the chromosomes are followed by the green fluorescent protein or red fluorescent protein, so as to trace the protein expression. Also, the protein expression is traced in cell passage culture.

### High detection sensitivity

The cell model constructed in the present invention belongs to a tool cell line that does not express cytokines such as IL-6 and TNF-α without the exposure to LPS. In cells from other sources, such as human monocytes, there exists the background expression of cytokines (such as IL-6 and TNF-α) even without the exposure to LPS, and this background expression may have the impact on the subsequent detection, however, in the cell model of the present invention, there exists no expression of cytokines, even background expression, without the exposure to LPS. Also, some gene engineering products may contain substances capable of promoting cell proliferation, and the cell proliferation at different levels will have the impact on uncertainty in detection, however, for the cell model of the present invention, there is no such risk. The lowest detection limit for LPS of the cell model of the present invention can reach 0.005EU/mL, while the lowest detection limit of the Limulus amebocyte lysate endotoxin test method is 0.025EU/mL, indicating that the sensitivity of the present invention is far better than that of the Limulus amebocyte lysate endotoxin test method.

### Overcoming the problems in the existing pyrogen detection

Limulus polyphemus is a national second class protected animal and its blood is rare, thus, the use of Limulus amebocyte lysate may affect the preservation of Limulus polyphemus and the sustainable development of biological resources, and the Limulus amebocyte lysate products from different manufacturers may produce different results of pyrogen detection in the same one sample. The monocyte activation test method requires human blood, and the monocytes from different human blood sources have different response stability, resulting in poor stability of detection. With the insertion of the detected genes into the chromosomes and the stable passage of the genes, the cell model provided by the present invention is superior in repeatability of pyrogen detection, responsivity and stability, and overcomes the problems of the conventional methods and the new human monocyte activation test method.

### The present invention has the advantages as follows.

The present invention provides a cytologic model for detecting pyrogens in drugs and biological products (including gene engineering drugs), a construction method thereof, and a pyrogen detection method and kit. By inducing double-bond breakage in the specific positions of genomes using CRISPR/CAS9, performing directed knock-in of TLR4 and CD14-MD2 on two chromosomes in a cell line based on the principle of homologous recombination and repair, and tracing using green fluorescent protein GFP and red fluorescent protein RFP respectively, we successfully construct the TLR4/CD14/MD2 directed knock-in and fluorescence-traceable cell model; upon the exposure to LPS, the release of IL-6 and TNF-a cytokines in the cell model can be detected by ELISA, Western Blot, mass spectrometry and magnetic bead method; and the cell model has good stability and high sensitivity, and the lowest detection limit can reach 0.005EU/mL, which is far lower than that of the Limulus amebocyte lysate endotoxin test method (0.025EU/mL).

### Brief Description of the Drawings

Fig. 1A is the plasmid profile of pMD19-T ligated with TLR4 in a preferred embodiment of the present invention.
Fig. 1B is the plasmid profile of pMD19-T ligated with CD14 in a preferred embodiment of the present invention.
Fig. 1C is the plasmid profile of pMD19-T ligated with MD2 in a preferred embodiment of the present invention.
Fig. 2A is the plasmid profile of transient expression vector pCAG-TLR4-GFP constructed by TLR4 and pMD19-T in a preferred embodiment of the present invention.
Fig. 2B is the plasmid profile of transient expression vector pCAG-CD14-GFP constructed by CD14 and pCAG-GFP in a preferred embodiment of the present invention.
Fig. 2C is the plasmid profile of transient expression vector pCAG-MD2-GFP constructed by MD2 and pCAG-GFP in a preferred embodiment of the present invention.
Fig. 3 shows a vector construction strategy of directed knock-in of TLR4 in a preferred embodiment of the present invention.
Fig. 4 schematically shows the principle of directed knocking of TLR4 into cells in a preferred embodiment of the present invention.
Fig. 5A is a picture of white light exposure after directed knocking of TLR4 into cells in a preferred embodiment of the present invention.
Fig. 5B is a picture of green fluorescence excited after directed knocking of TLR4 into cells in a preferred embodiment of the present invention.
Fig. 6 shows a vector construction strategy of directed knock-in of MD2-CD14 in a preferred embodiment of the present invention.
Fig. 7 schematically shows the principle of directed knocking of MD2-CD14 into cells in a preferred embodiment of the present invention.
Fig. 8 is a diagram of fluorescence detection after directed knocking of MD2-CD14 into cells in a preferred embodiment of the present invention.
Fig. 9 is a diagram of fluorescence expression conditions of four cell lines in a preferred embodiment of the present invention.
Fig. 10 is a diagram of expression conditions of four cell lines observed under confocal microscopy in a preferred embodiment of the present invention.
Fig. 11A schematically shows the strategy of PCR identification of TLR4 knocked into four cell lines in a preferred embodiment of the present invention.
Fig. 11B is an electrophoresis diagram of PCR results of Fig. 11A.
Fig. 11C schematically shows the strategy of PCR identification of CD14/MD2 knocked into four cell lines in a preferred embodiment of the present invention.
Fig. 11D is an electrophoresis diagram of PCR results of Fig. 11C.
Fig. 12 shows Western blot detection results of protein expression of four cell lines in a preferred embodiment of the present invention.
Fig. 13A shows ELISA detection of IL-6 release from four cell lines upon LPS exposure in a preferred embodiment of the present invention.
Fig. 13B shows ELISA detection of TNFα release from four cell lines upon LPS exposure in a preferred embodiment of the present invention.
Fig. 14 shows ELISA detection of IL-6 release from 293T/TLR4/CD14/MD2 cell lines upon LPS exposure over time in a preferred embodiment of the present invention.
Fig. 15 shows ELISA detection of IL-6 release amounts over different cell counts of 293T/TLR4/CD14/MD2 cell lines upon LPS exposure in a preferred embodiment of the present invention.
Fig. 16 shows ELISA detection of IL-6 release sensibility of 293T/TLR4/CD14/MD2 cell lines upon exposure to different amounts of LPS in a preferred embodiment of the present invention.
Fig. 17A shows Western blot assay of TNF-α response of 293T/TLR4/CD14/MD2 cell lines upon LPS exposure in a preferred embodiment of the present invention.
Fig. 17B shows Western blot assay of IL-6 response of 293T/TLR4/CD14/MD2 cell lines upon LPS exposure in a preferred embodiment of the present invention.

### Detailed Description of the Invention

The technical solution of the present disclosure will be further described below with reference to particular embodiments, however, these embodiments are not intended to limit the scope of the present disclosure in any way.

### Instruments and Materials

1. Restriction endonucleases are all purchased from Fermentas , company, Canada.
2. pMD19-T is purchased from Takara Biotechnology (Dalian) Co. Ltd.
3. pCAG-GFP, pCAG-RFP, pX330, pMCS.DT-A and pAAV-CAG-RFP are all purchased from Addgene Company.
4. Human monocyte isolation kit is purchased from Tianjin HaoYang Biologicals Company.
5. RPMI1640 medium (serum-free) and DMEM medium (serum-free) are purchased from GIBCO Company, and fetal calf serum from Hyclone Company.
6. HEK293T cell line is purchased from Cell Bank of the Chinese Academy of Sciences (Shanghai, China).
7. Sequencing: using general or specific primers, completed by Beijing Aokedingsheng Biotechnology (Dalian) Co. Ltd.

It should be noted that the portions related to detail steps that are not provided in the disclosure are all implemented by the conventional molecular biological methods or according to the reagent specifications.

### Example 1 Construction of a cell model

### 1. Preparation of human peripheral blood monocyte cDNA

### 1.1 Isolation and culture of human peripheral blood monocytes

5mL fresh anticoagulant blood from a healthy person was collected, human peripheral blood monocytes were extracted according to the specification of the human monocyte isolation kit and then added into RPMI1640 medium, the resulting mixture was washed and centrifuged once at 1500r/min for 10min, and the supernatant was abandoned. The cells were re-suspended with RPMI1640 medium containing 10% fetal calf serum (the medium was added 3-4mL per 1 mL blood sample), after cell counting, the cell density was adjusted until reaching 2×10⁷/mL, the suspension was cultured in a 10cm² dash, the dash was placed in 5% CO₂ incubator at 37°C for 2-3h, and the supernatant was abandoned, to obtain adherent human mononuclear cells. Then 10µL cell suspension that was diluted according to the dilution rate required was sucked and mixed with 10µL Trypan blue dye solution (0.4%), and after viable cell counting, the cell density was controlled at about 2 × 10⁷/mL.

### 1.2 Preparation of human peripheral blood monocyte cDNA

The cell culture solution of human peripheral blood monocytes prepared in step 1.1 was abandoned, the human peripheral blood monocytes were rinsed with PBS for three times and then added into each well of a 6-well plate, 200µL TRIzol Reagent (Gibco BRL) was added into each well, followed by repeated purging for cell lysis. Lysed cells were harvested and loaded in a 1.5mL centrifuge tube, and the centrifuge tube was placed at room temperature for 5min. 0.1mL Chloroform was added, followed by violent oscillation for 15sec and room temperature standing for 2-3min. 12,000g Cell suspension was centrifuged at 4°C for 15min, the water phase was sucked out and transferred to another 1.5mL centrifuge tube. 250µL Isopropanol was added, followed by thorough mixing and room temperature standing for 10min. 12,000g Cell suspension was centrifuged at 4°C for 5 10min for RNA precipitation, 1mL 75% ethanol was added for washing precipitation, and the precipitates was collected and dried in the air for 5-10min. A proper amount of RNase-free DEPC water was added for RNA dissolution in a 55-60°C water bath for 10min until RNA was fully dissolved. The concentration (OD₂₆₀) and purity (OD₂₆₀/OD₂₈₀) of the RNA sample were determined using an UV spectrophotometer, and then the RNA concentration was adjusted to 1µg/µL.

The RNA sample was denaturized under heating in a 70°C water bath for 5min, and then placed on ice for 5min. The reverse transcription reaction was carried out according to the following reaction systems, with the total reaction volume being 20µL.

**Table 1 RT reaction systems**

| | |
|---|---|
| M-MLV 5 ×reaction buffer | 4µL |
| dNTP(5mM) | 2µL |
| DTT(0.1M) | 2µL |
| random primer(20µM) | 5µL |
| RNasin RNase inhibitor (40U/µL) | 0.5µL |
| M-MLV reverse transcriptase (200U/µL) | 1µL |
| RNA(1 µg/µL) | 5.5µL |

The reagents were thoroughly mixed and inactivated at 37°C for 60min and then 70°C for 10min, water was added until reaching 100µL, and the resulting reaction system was stored at -20°C for later use.

### 2. Vector construction and identification of TLR4, CD14 and MD2 genes

### 2.1 Construction and identification of TLR4, CD14 and MD2 prokaryotic expression vectors

### 2.1.1 Acquisition of TLR4, CD14 and MD2 gene DNA fragments

mRNA sequences of human genome TLR4, CD14 and MD2 were obtained by NCBI search. RT-PCR primers were designed using Primer premier5.0 software with reference to related documents, restriction endonuclease recognition sequence EcoRI was introduced at 5' terminals of MD2 and CD14 upstream primers respectively and restriction endonuclease recognition sequence Kpnl and protection bases were introduced at 5' terminals of downstream primers, restriction endonuclease recognition sequence Kpnl was introduced at 5' terminals of TLR4 upstream primer and restriction endonuclease recognition sequence Smal and protection bases were introduced at 5' terminals of downstream primer, which were synthesized by Beijing Aokedingsheng Biotechnology Co. Ltd. The number of cycles was determined by the cycle curve diagram, to make the PCR process in geometric growth stage. Wherein, the enzyme digestion loca used were those not contained in the corresponding genes.

**Table 2 PCR primers sequences and PCR product lengths**

| Genes | Primer Sequences (5'-3') | Products(bp) |
|---|---|---|
| MD2-F(SEQ ID No.1) | GCGAATTCCATGTTACCATTTCTGTT | |
| MD2-R(SEQ ID No.2) | CCGGTACCTTGCAATTTGAATTAGGTT | 482 |
| CD14-F(SEQ ID No.3) | TAGAATTCATGGAGCGCGCGTCCTGCTTG | |
| CD14-R(SEQ ID No.4) | TTGGTACCTCGAAGGCAAAGCCCCGGG | 1127 |
| TLR4-F(SEQ ID No.5) | TCGGTACCGAGCTCGGATCCATGATGT | |
| TLR4-R(SEQ ID No.6) | ATCCCGGGTGCTCACCATCTCGAGGATA | 2519 |

**Table 3 PCR reaction systems**

| | |
|---|---|
| cDNA or plasmid template | 2µL |
| Sense primer (10µM) | 1µL |
| Anti-sense primer (10µM) | 1µL |
| 2×GoldStar BestMaster Mix | 10µL |
| Adding RNase-free Water up to 20µL | |

**Table 4 PCR reaction conditions**

| Genes | Pre-denaturation | Denaturation | Annealing | Extension | Number of cycles |
|---|---|---|---|---|---|
| MD2 | 95°C,10min | 95°C,45sec | 41°C, 30sec | 72°C, 1min | 35 |
| CD14 | 95°C,10min | 95°C,90s | 62°C, 1min | 72°C, 2min | 35 |
| TLR4 | 95°C,10min | 95°C,90s | 62°C, 2min | 72°C, 3min | 35 |

The reaction product was identified by agarose gel electrophoresis, and the identification result showed that the TLR4 gene was located around 2500bp, the CD14 gene around 1100bp, the MD2 gene around 500bp, in line with expectations. The TLR4 gene, CD14 gene and MD2 gene were extracted using agarose gel DNA extraction kit respectively and stored at -20°C.

### 2.1.2 Construction and identification of TLR4, CD14 and MD2 gene prokaryotic expression vectors

The extracted DNA fragments were ligated to a pMD19-T cloning vector respectively, with TLR4 being inserted between Kpn I and Sma I, CD14 between EcoR I and Kpn I and MD2 between EcoR I and Kpn I, to obtain there cloning vectors, and the cloning vectors were transformed into Escherichia coli DH5α, followed by amplification, plasmid extraction, enzyme digestion and electrophoresis identification, to obtain positive clones.

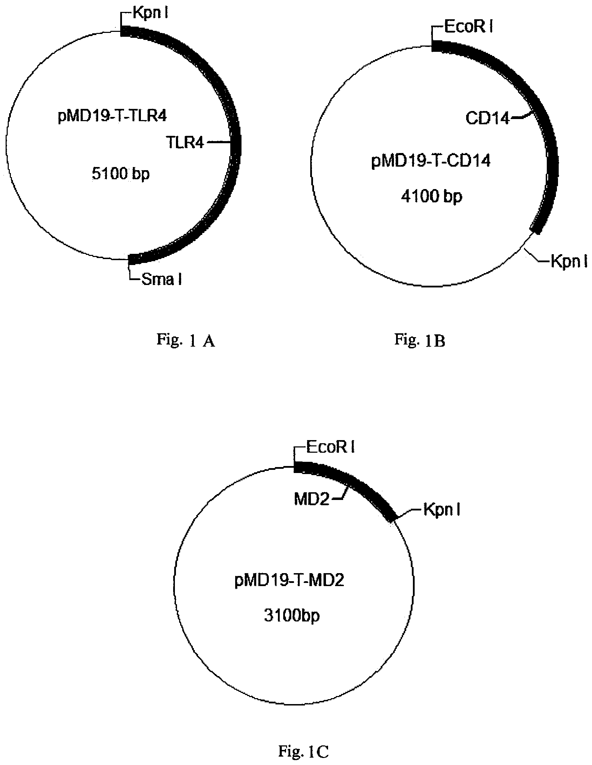

The obtained positive clones, named as pMD19-T-TLR4 (shown as Fig. 1A), pMD19-T-CD14 (shown as Fig. IB), pMD19-T-MD2 (shown as Fig. 1C), were sequenced, and the matching gene clones were found in Pubmed gene library.

### 2.2 Construction and identification of TLR4, CD14 and MD2 eukaryotic expression vectors

### 2.2.1 Construction and identification of TLR4, CD14 and MD2 eukaryotic expression vectors

The pMD19-T-TLR4 positive clone plasmid and pCAG-GFP eukaryotic expression vector plasmid that are determined having correct sequences were digested with both Smal and Kpnl, the restriction fragments were separated by electrophoresis, and the target fragments and vector arms were extracted.

The TLR4 fragment was ligated with the pCAG-GFP fragment, the ligation product was transformed into Escherichia coli DH5α, followed by amplification, plasmid extraction, enzyme digestion and electrophoresis identification, to obtain positive clones.

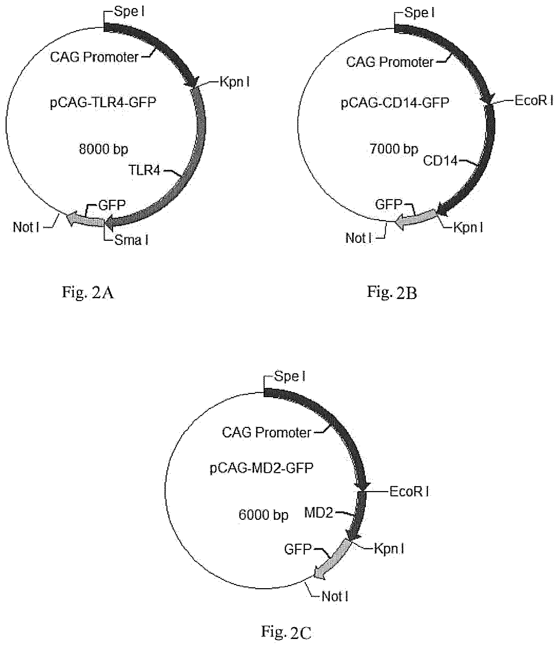

The obtained recombinant positive plasmid, named as pCAG-TLR4-GFP (shown as Fig. 2A), was sequenced, and the matching gene clones were found in Pubmed gene library.

The construction method of the MD2 and CD14 eukaryotic expression vectors were substantially the same as that of the TLR4 gene expression vector. The pMD19-T-MD2 and pMD19-T-CD14 positive clone plasmids that are determined having correct sequences and the pCAG-GFP eukaryotic expression vector plasmid were digested with both restriction endonucleases EcoRI and Kpnl and ligated, the monoclone was extracted after transformation and then cultured, and the plasmid was extracted in a small amount and then subjected to double-enzyme digestion and identification, after agarose gel electrophoresis and observation, the positive clones that were correctly inserted with the selected target fragments and had the desired sizes, named as pCAG-MD2-GFP (shown as Fig. 2B) and pCAG-CD14-GFP (shown as Fig. 2C) respectively, the positive clones were sequenced, and Blast was performed in Pubmed gene library, to determine whether the gene clones were matched.

### 3. Construction of TLR4 directed knocked-in cell model by CRISPR/Cas9 technology

### 3.1 Determination of knock-in targets

The DNA sequence of PPPP1R12C gene of human chromosome 19 was obtained by NCBI search in Nucleotide library. Human PPPP1R12C gene was located in 19q13.42, with GeneID: 54776. The full length of genome DNA was 26688bp, the first exon sequence was 1-378bp and the first intron sequence was 378-4806 bp, the first intron sequence of PPPP1R12C gene was input into the website: http://crispr.mit.edu/ to evaluate a property CRISPR knock-in target, and a property guideRNA target was obtained according to the result provided by the website. The sgRNA locus was located in 1849bp, and the long- and short-arms at about 1000bp upstream and about 1000bp downstream of the 1849bp sgRNA locus were amplified using the primers that were designed using Primer premier5.0 software.

In this example, the first intron position of genome DNA sequence of chromosome 19 was selected as directed knock-in site of TLR4 gene, and it has been confirmed that the insertion of a foreign gene in this position has no impact on the physiological functions of cells themselves. The TLR4 gene may also inserted in other chromosome loci that have no impact on the physiological functions of cells, in different genes, with the design of target primers being changed accordingly.

### 3.2 Design and construction of CRISPR/Cas 9 plasmid

### 3.2.1 Construction of CRISPR/Cas 9 plasmid

A target sgRNA sequence was synthesized using pX330 plasmid as skeleton, and Bbsl digestion sites were introduced at both ends. The sgRNA synthetic sequence was shown in Table 5.

**Table 5 PPPP1R12C-targeted sgRNA synthetic sequences**

| Primer Sequences (5'-3') | |
|---|---|
| Sense(SEQ ID No.7) | GATCGGGGGCCACUAGGGACAGGAUGTTTTAGAG |
| Anti-sense(SEQ ID No.8) | CTAGCTCTAAAACAUCCUGUCCCUAGUGGCCCCC |

The pX330 plasmid was digested with Bbs I, the restriction fragments were separated by electrophoresis, and the single pX330 restriction fragments were extracted. The complementary two sgRNA fragments were annealed and phosphorylated, and ligated to the linearized pX330 plasmid that has completed enzyme digestion. The ligation product was transformed into Escherichia coli DH5α, followed by amplification, plasmid extraction and single Bbsl enzyme digestion; and after enzyme digestion and identification, the electrophoretic examination was carried out to determine whether the inserted fragments exist, obtaining a recombinant positive plasmid, named as pPsg-Cas9, which was sequenced subsequently.

### 3.2.2 Construction of targeting vectors

Genome DNA of cell line HEK 293T (293T for short) was selected, and the sequences of about 1000bp at the upstream and downstream of sgRNA locus of PPPP1R12C gene were selected as long- and short-arms of targeting vectors. Primers were designed using Primer premier5.0 software, as shown in Table 6. The long- and short-arms were subjected to PCR and gel extraction and ligated to the pMD19-T vector, the ligation product was transformed, identified and sequenced, to obtain a pMD19-T-long arm plasmid and a pMD19-T arm plasmid.

**Table 6 PPPP1R12C gene long- and short-arm primers and product lengths**

| Name | Primer Sequences (5'-3') | Products(bp) |
|---|---|---|
| long arm-s(SEQ ID No.9) | ACTTTTATCTGTCCCCTCCACCC | |
| long arm-a(SEQ ID No.10) | CATCCCTGTGAAAGATGCCTGAG | 1315 |
| short arm-s(SEQ ID No.11) | TCTTCCGCATTGGAGTCGCTTTA | |
| short arm-a(SEQ ID No.12) | GTAAGCTTGAGGGGACAGATAAAAGTA | 1172 |

The long- and short-arms were digested with enzymes and ligated to the pMCS.DT-A targeting vector, by the following steps of: subjecting the pMCS.DT-A plasmid and pMD19-T-long arm plasmid that was determined having correct sequence to double enzyme digestion, electrophoresis and extraction, ligating the long arm fragment and vector fragment using T4 ligase, transforming Escherichia coli DH5α with the ligation product. The enzyme digestion, electrophoresis identification and sequencing identification were performed on the recombinant plasmids, to obtain a DTA-long arm; and the DTA-long arm plasmid and pMD19-T-short arm plasmid that were determined having correct sequences were digested with both Sal I and HindIII, separated by electrophoresis, extracted and ligated with the short-arm fragment and DTA-long arm restriction fragment using T4 ligase, and the ligation product was transformed into Escherichia coli DH5α. The enzyme digestion, electrophoresis identification and sequencing identification were performed on the recombinant plasmids, to obtain a DTA-long arm-short arm which is sequenced subsequently.

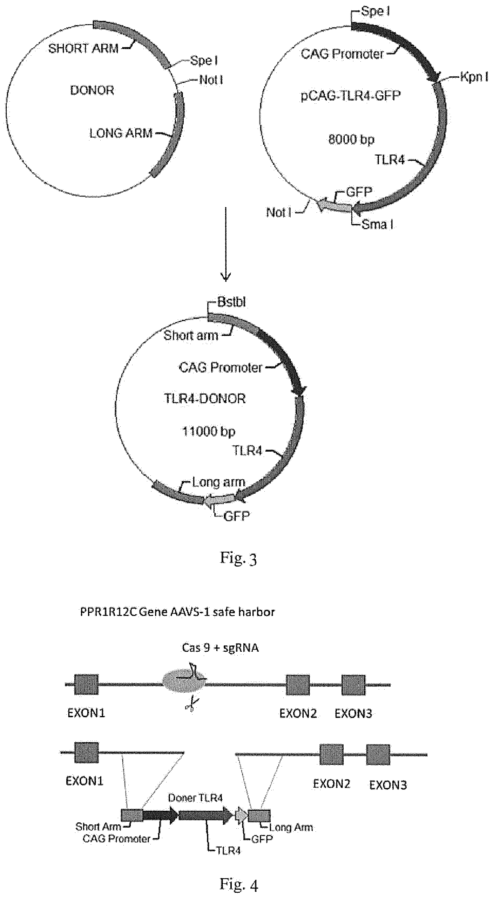

The DTA-long arm-short arm plasmid (DONOR) and pCAG-TLR4-GFP plasmid were digested with both Sal I and HindIII, separated by electrophoresis, extracted and ligated with the CAG-GFP-TLR4 fragment and DTA-long arm-short arm restriction fragment using T4 ligase, and the ligation product was transformed into Escherichia coli DH5α. The enzyme digestion, electrophoresis identification and sequencing identification were performed on the recombinant plasmid, to obtain a DTA-short arm-CAG-TLR4-GFP-longarm targeting vector (TLR4-DONOR), in which the construction strategy is shown as Fig. 3, and the principle of directed knocking of TLR4 into cells is schematically shown as Fig. 4.

### 3.2.3 Electrotransfection of targeting vectors and CRISPR/Cas9 plasmid

The cells of 293T cell line, 24hr before electrotransfection, were inoculated in an amount of 0.5-1.0×10⁵ into a 6-well plate, with the degree of cell confluence reaching 90-95% during electrotransfection; the targeting vector DTA-shortarm-CAG-TLR4-GFP-longarm was linearized, at the short-arm 5' terminal of homologous recombination arm, by single BstBI digestion; the cells were rinsed with PBS and digested with pancreatin, the digestion product was centrifuged at 800rpm for 3min, and the cells were harvested; and the 293T cells were re-suspended using an electrotransfection buffer (DMEM-free), with the cell density being adjusted to 1×10⁷/mL. An electroporation cup (4mm) was selected for electroporation of 300µL cells, 25ng targeting vector and 25ng pPsg-Cas plasmid each time. The electroporation operation was carried out at room temperature at 260v for 30ms, then the cells were inoculated into the 6-well plate, with the culture medium (2mL) being supplemented, and cultured at 37°C. The fluorescence condition was observed 48hr after cell electrotransfection.

### 3.2.4 Selection of monoclones by limiting dilution method

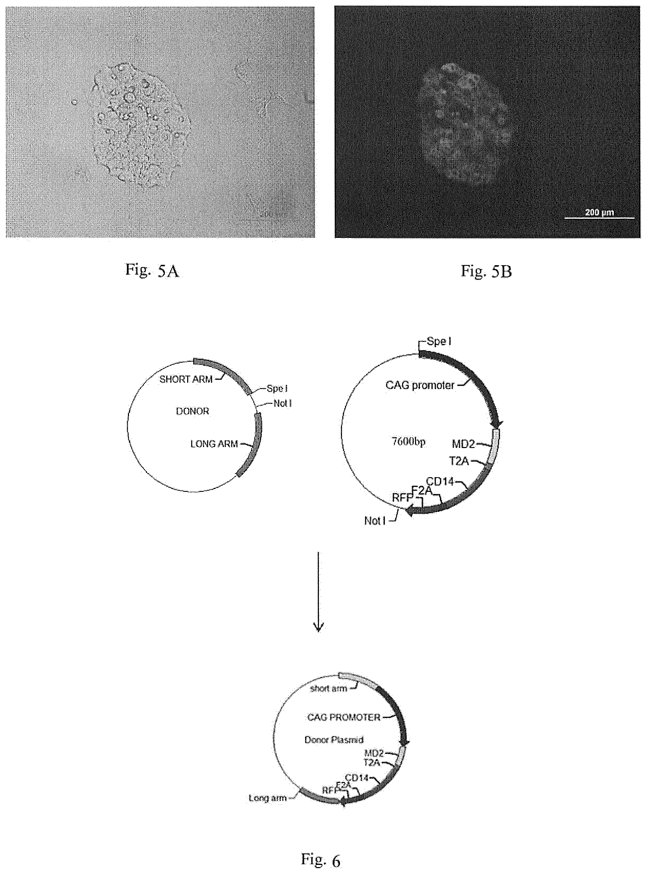

Cells that grow well were harvested, and a cell suspension was prepared. The cell count of the cell suspension was accurately calculated by cell counting method; the cells were diluted by gradient dilution method to prepare a 10/mL cell suspension, namely, one cell/0.1 mL; the cell suspension was inoculated into a 96-well plate, with the amount of 0.1 mL/well; the 96-well plate was placed in a 5% CO2 incubator at 37°C and taken out for observation 4 days later, and the observation results were recorded and analyzed; and the monoclone-growing wells where monoclones grow well and have strong green fluorescence were selected, the monoclones were transferred into a 24-well plate for cloning and culture or enlargement culture and then subjected to multiple passage, to obtain a cell clone capable of stably expressing green fluorescent proteins, named as cell clone 293T/TLR4, and it can be seen from Fig. 5A and Fig. 5B that when the cell clone 293T/TLR4 was exposed to white light, no fluorescence was observed, while when exposed to exciting light, green fluorescence was observed.

### 4. Construction of CD14-MD2 directed knocked-in cell model by CRISPR/Cas9 technology

### 4.1 Construction of pCAG-MD2-2A-CD 14-2A-RFP plasmid

The RFP fragment was subjected to PCR amplification using pAAV-CAG-RFP as template, and F2A sequence (shown as Table 7) and AgeI digestion site were introduced at the same time of primer design at 5'-terminal, and Not I digestion site was introduced at 3'-terminal. The pCAG-CD14-GFP plasmid was digested with AgeI and Not I to cut off the green fluorescent protein (GFP) fragment and ligated to a F2A-RFP fragment, with the primer sequences shown as Table 8; and after enzyme digestion and sequencing identification, a pCAG-CD14-F2A-RFP plasmid was obtained and transformed into 293T cells by transient transfection, followed by analysis of fluorescence expression; the MD2 fragment was subjected to PCR amplification, and T2A sequence (shown as Table 7) and ageI digestion site were introduced at 3'-terminal, and the pCAG-CD14-F2A-RFP plasmid was digested with Kpn I and ligated to the MD2-T2A fragment by homologous recombination method using abm cloning kit, with the primer sequences shown as Table 8; and after enzyme digestion and sequencing identification, a pCAG-MD2-T2A-CD14-F2A-RFP plasmid was obtained.

**Table 7 Sequences of F2A and T2A**

| Sequence names | Sequence (5'-3') | Lengths |
|---|---|---|
| F2A (SEQ ID No.13) | CAGCTGTTGAATTTTGACCTTCTTAAGCTT | 54bp |
| | GCGGGAGACGTCGAGTCCAACCCT | |
| T2A(SEQ ID No.14) | GAGGGCAGAGGAAGTCTTCTAACATGCGGT | 54bp |
| | GACGTGGAGGAGAATCCCGGCCCT | |

**Table 8 F2A-RFP and MD2-T2A primers and product lengths**

| Name | Primer Sequences (5'-3') | Products(bp) |
|---|---|---|
| F2A-RFP-sense | CGGGCCCGGGATCCACAGCTGTTGAATT | |
| (SEQ ID No.15) | TTGACCTTCTTAAGCTTGCGGGAGACGT | |
| | CGAGTCCAACCCTATGGCCTCCTCCGAG | 739 |
| | GAC | |
| F2A-RFP-antisense | CTGAGGAGTGCGGCCCTACAGGAACAG | |
| (SEQ ID No.16) | GTGGTGGC | |
| MD2-T2A-sense | TTTGGCAAAGAATTCATG | |
| (SEQ ID No.17) | | |
| MD2-T2A-antisense | ACGTCACCGCATGTTAGAAGACTTCCTC | 576 |
| (SEQ ID No.18) | TGCCCTCCTGATTTGAATTAGGTTG | |

### 4.2 Determination of knock-in targets

The genomic DNA sequence of CCR5 gene of human chromosome 3 was obtained by NCBI search in Nucleotide library. The first intron sequence of CCR5 gene was input into the website: http://crispr.mit.edu/ to evaluate a property CRISPR knock-in target, and a property guideRNA target was obtained according to the result provided by the website.

In this example, the first intron position of genome DNA sequence of chromosome 3 was selected as directed knock-in site of CD14 and MD2 genes, and it has been confirmed that the insertion of a foreign gene in this position has no impact on the physiological functions of cells themselves. The CD14 and MD2 genes may also inserted in other chromosome loci that have no impact on the physiological functions of cells, in different genes, with the design of target primers being changed accordingly.

### 4.3 Design and construction of CRISPR/Cas 9 plasmid

### 4.3.1 Construction of CRISPR/Cas 9 plasmid

A complementary target sgRNA sequence was synthesized using pX330 plasmid as skeleton, and Bbsl digestion sites were introduced at both ends. The sgRNA synthetic sequence was shown in Table 9.

**Table 9 CCR5-targeted sgRNA synthetic sequences**

| | Primer Sequences (5'-3') |
|---|---|
| Sense(SEQ ID No.19) | GATCGUGAGGGCUUAACACAGUGCCGTTTTAGAG |
| Anti-sense(SEQ ID No.20) | CTAGCTCTAAAACGGCACUGUGUUAAGCCCUCAC |

The pX330 plasmid was digested with Bbs I, the restriction fragments were separated by electrophoresis , and the single pX330 restriction fragments were extracted. The complementary two sgRNA fragments were annealed and phosphorylated, and ligated to the linearized pX330 plasmid that has completed enzyme digestion. The ligation product was transformed into Escherichia coli DH5α, followed by amplification, plasmid extraction and single Bbsl digestion; and after enzyme digestion and identification, the electrophoretic examination was carried out to determine whether the inserted fragments exist, obtaining a recombinant positive plasmid, named as pPsg-Cas, which was sequenced subsequently.

### 4.3.2 Construction of CCR5 gene targeting vectors

Sequences of about 1000bp at the upstream and downstream of sgRNA locus of CCR5 gene were selected as long- and short-arms of targeting vectors. Primers were designed using Primer premier5.0 software, as shown in Table 10. The long- and short-arms were subjected to PCR and gel extraction and ligated to the pMD19-T vector, and the ligation product was transformed, identified and sequenced.

**Table 10 PCR long- and short-arm sequences**

| Name | Primer Sequences (5'-3') | Products(bp) |
|---|---|---|
| long arm-s(SEQ ID No.21) | TGGTAACAGTTAGCCACTT | 1373 |
| long arm-a(SEQ ID No.22) | GATTCTTCATCTTCCCTTT | |
| short arm-s(SEQ ID No.23) | AACACCTCCTACTATTTACTG | 1072 |
| short arm-a(SEQ ID No.24) | AGCCCTCAACATGCAATTTCTC | |

The long- and short-arms were digested with enzymes and ligated to the pMCS.DT-A targeting vector, for more details, referring to step 3.2.2, in which the short arm was ligated between Sal I and HindIII of the pMCS.DT-A vector, and the long arm was ligated between Sac II and Sac I, finally obtaining DTA-long arm-short arm.

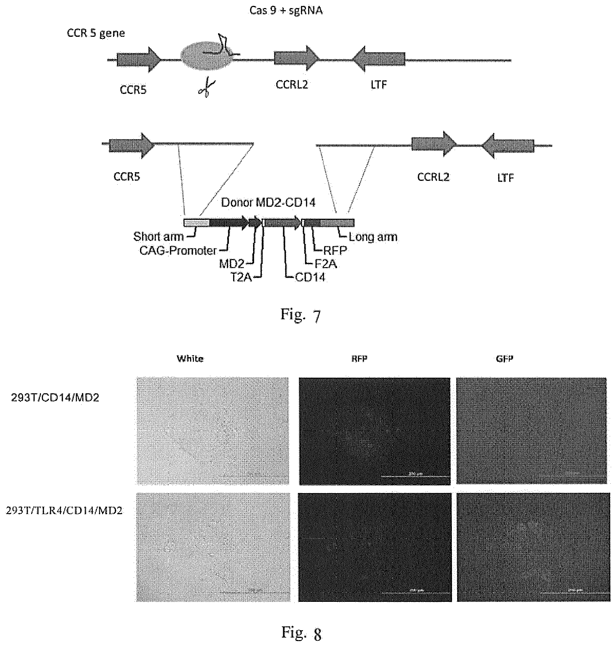

Then, pCAG-MD2-T2A-CD14-F2A-RFP plasmid was digested with enzymes and ligated to the DTA-long arm-short arm vector, for more details, referring to step 3.2.2, finally obtaining a DTA-short arm-CAG-MD2-T2A-CD14-F2A-RFP-long arm targeting vector, and the construction Strategy was shown as Fig. 6, and the principle of directed knocking of MD2 and CD14 into cells was schematically shown as Fig. 7.

### 4.3.3 Electrotransfection of targeting vectors and CRISPR/Cas9 plasmid

The targeting vector and pCsg-Cas were electrotransfected into 293T and 293T/TLR4 cells with reference to step 3.2.3, and single clones were screened with reference to step 3.2.4, to obtain two clone cells providing red fluorescent proteins, namely 293T/CD14/MD2 cell and 293T/TLR4/CD14/MD2 cell, and as shown in Fig. 8, it can be seen that red fluorescence was observed in both cell clones. Wherein the 293T/CD14/MD2 cell served as the control and 293T/TLR4/CD14/MD2 cell served as the target cell line.

### 5 Identification of cell models

### 5.1 Fluorescence identification and confocal microscopic identification

### 5.1.1 Fluorescence identification

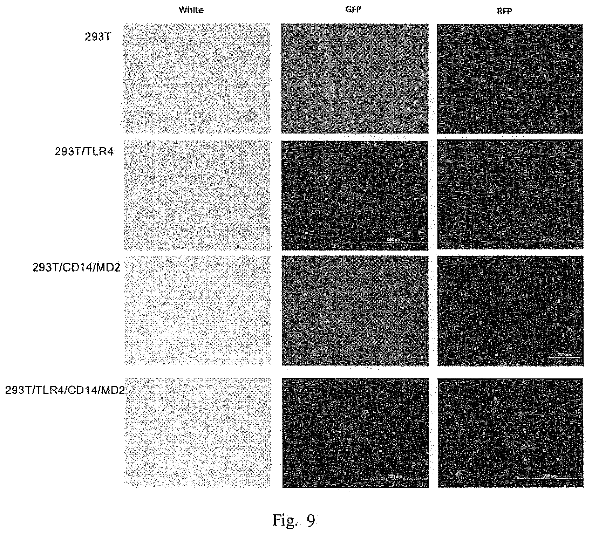

Four cell lines 293T, 293T/TLR4, 293T/CD14/MD2 and 293T/TLR4/CD14/MD2 were all cultured by 3 passages and then exposed to fluorescent light, as shown in Fig. 9. The results showed that no expression of green fluorescent protein or red fluorescent protein was observed in cell line 293T, stable expression of green fluorescent protein was observed in cell line 293T/TLR4, stable expression of red fluorescent protein was observed in cell line 293T/CD14/MD2, and stable expression of both green fluorescent protein and red fluorescent protein was observed in cell line 293T/TLR4/CD14/MD2.

### 5.1.2 Identification of confocal microscopy

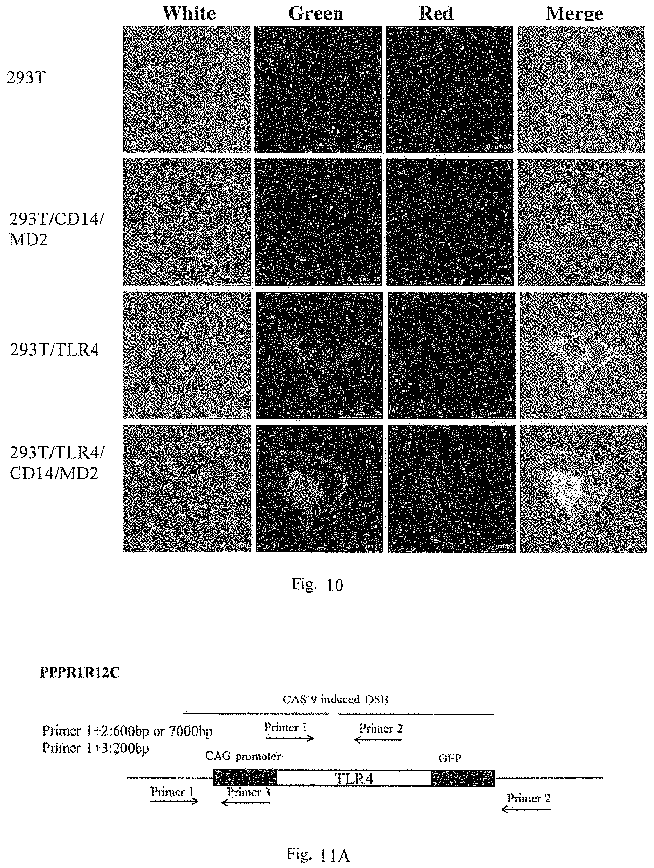

The above four cell lines were further identified by confocal microscopy, as shown in Fig. 10, it can be seen that no expression of green fluorescent protein or red fluorescent protein was observed in cell line 293T, stable expression of green fluorescent protein was observed in cell line 293T/TLR4, stable expression of red fluorescent protein was observed in cell line 293T/CD14/MD2, and stable expression of both green fluorescent protein and red fluorescent protein was observed in cell line 293T/TLR4/CD14/MD2. When Fig. 9 and Fig. 10 were overlapped, it showed that, for cell line 293T/TLR4/CD14/MD2 (Merge mode), the images of green fluorescence and red fluorescence were superimposed, while for cell lines 293T/TLR4 and 293T/CD14/MD2, the images of green fluorescence and red fluorescence were not superimposed, which was consistent with the results of fluorescence identification. Also, it can be seen that, for TLR4, the green fluorescence was mainly observed in cell membranes and cytoplasms, while for CD14-MD2, the red fluorescence was mainly observed in cytoplasms.

### 5.2 PCR identification

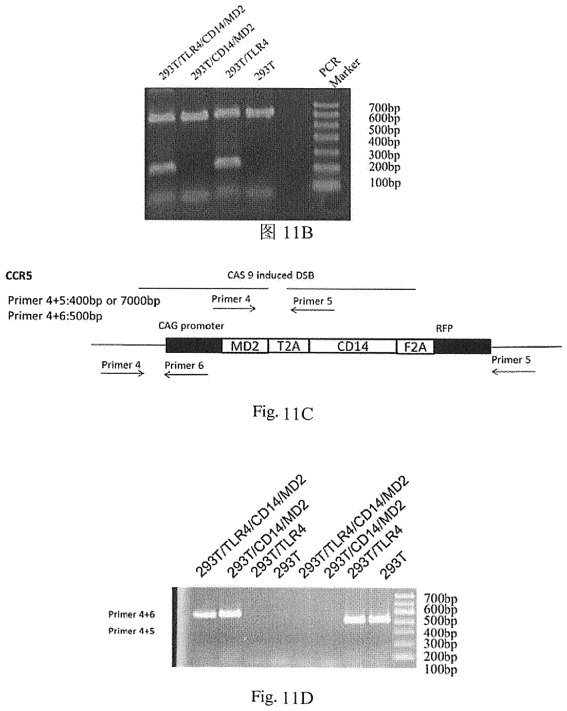

Genome DNA of cell lines 293T, 293T/TLR4, 293T/CD14/MD2 and 293T/TLR4/CD14/MD2 were extracted and analyzed using primers that were designed at different loci of the genomes to determine whether the knock-in operation was proper, the primer sequences being shown as Table 11. Wherein, fragments can be derived from the wild-type cell line by amplification using primer 1+primer2 and primer4+primer5, while no fragments can be derived by amplification using primer 1+primer 3 and primer 4+ primer 6. Fragments can be derived from the TLR4 knocked-in cell line by amplification using primer 1+primer 3, and fragments can be derived from the CD14-MD2 knocked-in cell line by amplification using primer 4+ primer 6. As shown from Fig. 11A to Fig. 11D, it can be determined by PCR identification that the knock-in loci of cell lines 293T/TLR4, 293T/CD14/MD2 and 293T/TLR4/CD14/MD2 were correct.

**Table 11 Primer sequences for PCR identification**

| Name | Primer Sequences (5'-3') |
|---|---|
| Primer 1(SEQ ID No.25) | TCTTCTTCCTCCAACCC |
| Primer 2(SEQ ID No.26) | GACCCAATATCAGGAGAC |
| Primer 3(SEQ ID No.27) | GGCTATGAACTAATGACCC |
| Primer 4(SEQ ID No.28) | CGGGAATGCTTTGTATC |
| Primer 5(SEQ ID No.29) | TGCCCGCTATGTCTAA |
| Primer 6(SEQ ID No.30) | ATGGGCTATGAACTAATGAC |

### 5.3 Western blot identification of protein expression

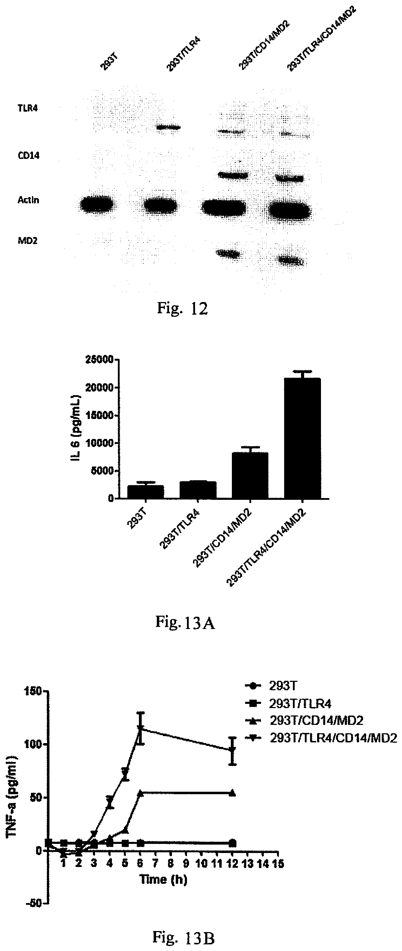

The four cell lines 293T, 293T/TLR4, 293T/CD14/MD2 and 293T/TLR4/CD14/MD2 were lysed for total protein extraction. The expression of TLR4, CD14 and MD2 proteins was identified by Western blot assay using Actin as internal standard. As shown in Fig. 12, no expression of TLR4, CD14 or MD2 was observed in cell line 293T, the expression of TLR4 was observed in cell line 293T/TLR4 with no expression of CD14 or MD2, the expression of TLR4, CD14 and MD2 was observed in cell line 293T/CD14/MD2, and the expression of TLR4, CD14 and MD2 was observed in cell line 293T/TLR4/CD14/MD2.

Based on the results of analysis, as endogenous TLR4 in cells was activated due to the presence of CD14/MD2, the expression of a few amount of TLR4 was observed in cell line 293T/CD14/MD2. Example 2 Detection of pyrogen marker LPS using the cell model

### 1 Identification of LPS response and detection of expression of IL-6 and TNFα in cell lines by ELISA assay

The cell lines 293T, 293T/TLR4, 293T/CD14/MD2 and 293T/TLR4/CD14/MD2 were exposed to LPS, and the release of IL-6 and TNFα cytokines in the supernatants were detected by ELISA assay, and the results showed that no response to LPS was observed in the cell lines 293T and 293T/TLR4, the release of IL-6 or TNFα was observed in the cell lines 293T/CD14/MD2 and 293T/TLR4/CD14/MD2 upon the exposure to LPS, and the release amount of IL-6 or TNFα the cell line 293T/CD14/MD2 was less than that in the cell line 293T/TLR4/CD14/MD2, as shown in Fig. 13A and Fig. 13B.

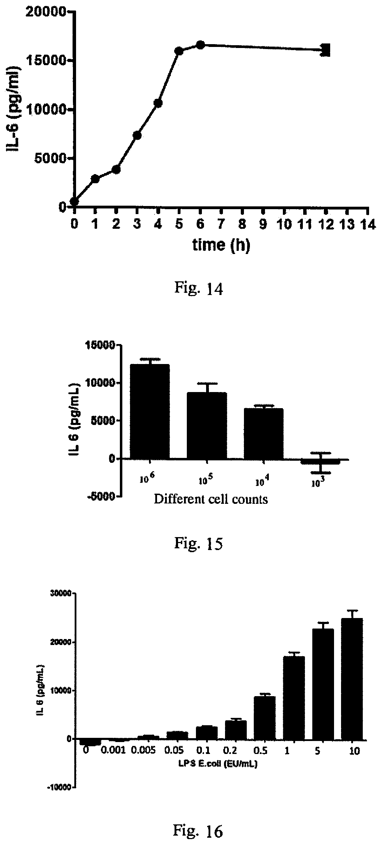

The cell line 293T/TLR4/CD14/MD2 was kept for the subsequent tests. The release of IL-6 upon the exposure to LPS was detected at different time points. It was found that the release of IL-6 peaked about 5-6h, as shown in Fig. 14. The release amounts of IL-6 were detected in the cell line 293T/TLR4/CD14/MD2 at different cell counts upon the exposure to 5EU/mL LPS for 6h, as shown in Fig. 15, it can be seen that the release of IL-6 can be detected in the cell line 293T/TLR4/CD14/MD2 at the cell count of 10⁴ upon the exposure to 5EU/mL LPS. The lowest detection limit of the cell line 293T/TLR4/CD14/MD2 upon the exposure to LPS was determined, and it can be seen from Fig. 16 that the release of IL-6 can be detected in the cell line 293T/TLR4/CD14/MD2 upon the exposure to 0.005EU/mL LPS, which was better than that of the Limulus amebocyte lysate endotoxin test method.

### 2 Identification of LPS response and detection of expression of IL-6 and TNFα in cell line 293T/TLR4/CD14/MD2 by Western blot assay

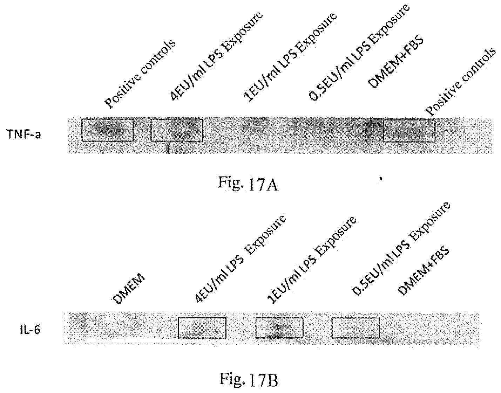

1×10⁶ 293T/TLR4/CD14/MD2 cells were exposed to high concentration LPS (4EU/mL), medium concentration LPS (1EU/mL) and low concentration LPS (0.5EU/mL), 1mL supernatants were collected 6h after culture and concentrated to 200µL, and the results of Western blot assay showed that the release of IL-6 and TNF-a were detected in the culture supernatants, as shown in Fig. 17A and Fig. 17B.

### 3. Identification of LPS response and detection of expression of IL-6 and TNFα in cell line 293T/TLR4/CD14/MD2 by mass spectrometry

After the cell line 293T/TLR4/CD14/MD2 was exposed to LPS for 5 hours, 1000µL culture supernatant was collected, purified, analyzed by matrix-assisted laser desorption/ionization time of flight mass spectrometry (MALDI. TOF-MS) and corrected, to provide the peptide mass fingerprinting of each protein point, the fingerprint pattern was treated using Mascot Distiller software to identify single isotope signal peak, to provide values of peptide fragment mass-to-charge ratio (m/z), the values were submitted to Mas-cot system and searched in IPI. HUMAN. v3.53 database, and the result was determined positive if Mascot score was more than 61 (P<0.05).

### 4. Identification of LPS response and detection of expression of IL-6 and TNFα in cell line 293T/TLR4/CD14/MD2 by colloidal gold strip assay

Colloidal gold strip assay works based on antigen-antibody reaction using nano-sized colloidal gold as tracer and detection agent, during detection, the cell line 293T/TLR4/CD14/MD2 was exposed to LPS for 5 hours (the test sample and culture method were the same as Western Blot and ELISA), the supernatant (50µL) of the culture liquid was collected and detected using colloidal gold strips for IL-6 and TNFα detection (commercially available or self-made), and the determination whether the expressions of IL-6 and TNFα were positive was carried out according to the results of the strips.

From the above examples, it can be seen that the cell model provided by the present invention is pyrogen-responsive, can detect endotoxins of Gram-negative bacteria and non-endotoxin derived contaminants, including biological or chemical entities associated with pathogen associated molecular patterns (PAMPs) and products of Gram-positive bacteria, Gram-negative bacteria, viruses and fungi and biological or chemical entities associated with processing technologies, has the lowest detection limit lower than the Limulus amebocyte lysate endotoxin test method, has stronger sensitivity, and has higher stability as compared to the method using human whole blood.

## Claims

1. A construction method of a cell model for detecting pyrogens, **characterized by** including the following steps of:
1) assembling TLR4 gene into an expression vector capable of expressing a green fluorescent protein, to construct a recombinant plasmid containing TLR4 gene, wherein the recombinant plasmid is pCAG-TLR4-GFP;
2) knocking the TLR4 gene in a directed manner into an intron of a gene on a chromosome of a cell line by the CRISPR/CAS9 method, to obtain a cell line capable of expressing the TLR4 gene stably;
3) segregating CD14 and MD2 genes from red fluorescent protein RFP gene using two different 2A peptides, wherein the two different peptides are F2A and T2A, to construct a recombinant plasmid containing CD14 and MD2 genes and capable of expressing a red fluorescent protein, wherein the recombinant plasmid is pCAG-MD2-T2A-CD14-F2A-RFP;
4) knocking the CD14 and MD2 genes in another intron of a gene on another chromosome than the chromosome on which the TLR4 gene is knocked-in in step 2) by the CRISPR/CAS9 method, to obtain a cell line capable of expressing the TLR4, CD14 and MD2 genes stably and capable of expressing green and red fluorescent proteins, wherein the knocked-in TLR4 gene is traced by the green fluorescent protein and wherein the knocked-in CD14 and MD2 genes are traced by the red fluorescent protein.

2. The construction method of claim 1, **characterized in that** the cell line is cell line HEK293T, HEK293 or NIH3T3.

3. The construction method of claim 1, **characterized in that** the position where the TLR4 gene is knocked-in in step 2) is the first intron of PPP1R12C gene of chromosome 19.

4. The construction method of claim 1, **characterized in that** the position where the CD14 gene is knocked-in in step 4) is the first intron of CCR5 gene of chromosome 3.

5. A cell model constructed by the construction method of any one of claims 1-4, **characterized in that** the cell model is obtained by knocking the TLR4 gene in the first intron of PPP1R12C gene of chromosome 19 and knocking the CD14 and MD2 genes in the first intron of CCR5 gene of chromosome 3 of the cell line HEK 293T.

6. The cell model of claim 5, **characterized in that** the systematic name of the cell model is human
embryonic kidney (HEK) cell variant 293T/TLR4/CD14/MD2, preserved in May 19, 2016 in China General Microbiological Culture Collection Center (CGMCC), Institute of Microbiology, Chinese Academy of Sciences, No.1 Beichen West Road, Chaoyang District, Beijing, with the preservation number CGMCC No. 12296.

7. A kit for detecting pyrogens, **characterized by** including 1) the cell model constructed by the construction method of any one of claims 1-4, or the cell model of claim 5 or 6, 2) a pyrogen standard sample, and 3) an IL6 and/or TNFα control sample.

8. The kit for detecting pyrogens of claim 7, **characterized in that** the pyrogen standard sample is LPS freeze-dried powder, the IL6 control sample is IL6 freeze-dried powder, and the TNFα control sample is TNFα freeze-dried powder.

9. A method for detecting pyrogens, **characterized by** including the following steps:
1) preparing a test sample solution and a pyrogen standard sample solution;
2) adding the test sample solution into a culture medium containing a cell model constructed by the construction method of any one of claims 1-4, or the cell model of claim 5 or 6, and culturing at 37°C for 5h;
3) collecting a supernatant of the culture product, and detecting the contents of IL-6 and/or TNF-α in the supernatant.

10. The method for detecting pyrogens of claim 9, **characterized in that** detecting the contents of IL-6 and/or TNF-α in step 3) is implemented by Western Blot, ELISA, gold standard method or mass spectrometry.

## Patentansprüche

1. Verfahren zur Konstruktion eines Zellmodells für den Nachweis von Pyrogenen, **dadurch gekennzeichnet, dass** es die folgenden Schritte beinhaltet:
1) Einbauen eines TLR4-Gens in einen Expressionsvektor, der in der Lage ist, ein grün fluoreszierendes Protein zu exprimieren, um ein rekombinantes Plasmid zu konstruieren, welches das TLR4-Gen enthält, wobei das rekombinante Plasmid pCAG-TLR4-GFP ist,
2) Inaktivieren des TLR4-Gens auf eine gezielte Weise in ein Intron eines Gens auf einem Chromosom einer Zelllinie durch das CRISPR/CAS9-Verfahren, um eine Zelllinie zu erhalten, die in der Lage ist, das TLR4-Gen stabil zu exprimieren,
3) Abtrennen der CD14- und MD2-Gene von dem Gen für rot fluoreszierendes Protein bzw. RFP unter Verwendung zweier unterschiedlicher 2A-Peptide, wobei die zwei unterschiedlichen Peptide F2A und T2A sind, um ein rekombinantes Plasmid zu konstruieren, das CD14- und MD2-Gene enthält und in der Lage ist, ein rot fluoreszierendes Protein zu exprimieren, wobei das rekombinante Plasmid pCAG-MD2-T2A-CD14-F2A-RFP ist,
4) Inaktivieren der CD14- und MD2-Gene in ein anderes Intron eines Gens auf einem anderen Chromosom als dem Chromosom, in welches das TLR4-Gen in Schritt 2) inaktiviert wurde, durch das CRISPR/CAS9-Verfahren, um eine Zelllinie zu erhalten, die in der Lage ist, die TLR4-, CD14- und MD2-Gene stabil zu exprimieren, und in der Lage ist, grün und rot fluoreszierende Proteine zu exprimieren, wobei das inaktivierte TLR4-Gen durch das grün fluoreszierende Protein markiert wird und wobei die inaktivierten CD14- und MD2-Gene durch das rot fluoreszierende Protein markiert werden.

2. Konstruktionsverfahren nach Anspruch 1, **dadurch gekennzeichnet, dass** die Zelllinie die HEK293T-, HEK293- oder NIH3T3-Zellinie ist.

3. Konstruktionsverfahren nach Anspruch 1, **dadurch gekennzeichnet, dass** die Position, an welcher das TLR4-Gen in Schritt 2) inaktiviert wird, das erste Intron des PPP1R12C-Gens von Chromosom 19 ist.

4. Konstruktionsverfahren nach Anspruch 1, **dadurch gekennzeichnet, dass** die Position, an welcher das CD14-Gen in Schritt 4) inaktiviert wird, das erste Intron des CCR5-Gens von Chromosom 3 ist.

5. Zellmodell, das mit dem Konstruktionsverfahren nach einem der Ansprüche 1-4 konstruiert wurde, **dadurch gekennzeichnet, dass** das Zellmodell durch Inaktivieren des TLR4-Gens in das erste Intron des PPP1R12C-Gens von Chromosom 19 und Inaktivieren der CD14- und MD2-Gene in das erste Intron des CCR5-Gens von Chromosom 3 der Zellelinie HEK 293T erhalten wird.

6. Zellmodell nach Anspruch 5, **dadurch gekennzeichnet, dass** der systematische Name des Zellmodells Menschliche embryonale Nieren- bzw. HEK-Zellvariante 293T/TLR4/CD14/MD2 lautet, hinterlegt am 19. Mai 2016 im China General Microbiological Culture Collection Center (CGMCC), Institute of Microbiology, Chinese Academy of Sciences, Nr. 1 Beichen West Road, Chaoyang District, Beijing, mit der Hinterlegungszahl CGMCC Nr. 12296.

7. Kit zum Nachweis von Pyrogenen, **dadurch gekennzeichnet, dass** es 1) das Zellmodell, das durch das Konstruktionsverfahren nach einem der Ansprüche 1-4 konstruiert wurde, oder das Zellmodell nach Anspruch 5 oder 6, 2) eine Pyrogen-Standardprobe, und 3) eine IL6- und/oder TNFα-Kontrollprobe beinhaltet.

8. Kit zum Nachweis von Pyrogenen nach Anspruch 7, **dadurch gekennzeichnet, dass** die Pyrogen-Standardprobe lyophilisiertes LPS-Pulver ist, die IL6-Kontrollprobe lyophilisiertes IL6-Pulver ist, und die TNFα-Kontrollprobe lyophilisiertes TNFα-Pulver ist.

9. Verfahren zum Nachweis von Pyrogenen, **dadurch gekennzeichnet, dass** es die folgenden Schritte beinhaltet:
1) Herstellen einer Testproben-Lösung und einer Pyrogen-Standardproben-Lösung,
2) Zugeben der Testproben-Lösung zu einem Kulturmedium, das ein Zellmodell, das durch das Konstruktionsverfahren nach einem der Ansprüche 1-4 konstruiert wurde, oder das Zellmodell nach Anspruch 5 oder 6 enthält, und Kultivieren bei 37 °C für 5 h,
3) Sammeln eines Überstands des Kulturprodukts und Nachweisen eines Gehalts an IL-6 und/oder TNF-α in dem Überstand.

10. Verfahren zum Nachweis von Pyrogenen nach Anspruch 9, **dadurch gekennzeichnet, dass** das Nachweisen des Gehalts an IL-6 und/oder TNF-α in Schritt 3) durch Western-Blotting, ELISA, Standardmethode oder Massenspektrometrie implementiert ist.

## Revendications

1. Procédé de construction d'un modèle cellulaire pour détecter des pyrogènes, **caractérisé en ce qu'**il inclut les étapes suivantes :
1) assemblage du gène TLR4 dans un vecteur d'expression capable d'exprimer une protéine fluorescente verte, pour construire un plasmide recombiné contenant le gène TLR4, lequel plasmide recombiné est pCAG-TLR4-GFP ;
2) invalidation du gène TLR4 d'une manière dirigée dans un intron d'un gène sur un chromosome d'une lignée cellulaire par le procédé CRISPR/CAS9, pour que soit obtenue une lignée cellulaire capable d'exprimer le gène TLR4 de façon stable ;
3) ségrégation des gènes CD14 et MD2 du gène de protéine fluorescente rouge RFP en utilisant deux peptides 2A différents, lesquels deux peptides différents sont F2A et T2A, pour construire un plasmide recombinant contenant les gènes CD14 et MD2 et capable d'exprimer une protéine fluorescente rouge, lequel plasmide recombinant est pCAG-MD2-T2A-CD14-F2A-RFP ;
4) invalidation des gènes CD14 et MD2 dans un autre intron d'un gène sur un chromosome autre que le chromosome sur lequel le gène TLR4 est invalidé dans l'étape 2) par le procédé CRISPR/CAS9, pour que soit obtenue une lignée cellulaire capable d'exprimer les gènes TLR4, CD14 et MD2 de façon stable et capable d'exprimer les protéines fluorescentes verte et rouge, lequel gène TLR4 invalidé est tracé par la protéine fluorescente verte et lesquels gènes CD14 et MD2 invalidés sont tracés par la protéine fluorescente rouge.

2. Procédé de construction selon la revendication 1, **caractérisé en ce que** la lignée cellulaire est la lignée cellulaire HEK293T, HEK293 ou NIH3T3.

3. Procédé de construction selon la revendication 1, **caractérisé en ce que** la position où le gène TLR4 est invalidé dans l'étape 2) est le premier intron du gène PPP1R12C du chromosome 19.

4. Procédé de construction selon la revendication 1, **caractérisé en ce que** la position où le gène CD14 est invalidé dans l'étape 4) est le premier intron du gène CCR5 du chromosome 3.

5. Modèle cellulaire construit par le procédé de construction de l'une quelconque des revendications 1 à 4, **caractérisé en ce que** le modèle cellulaire est obtenu par invalidation du gène TLR4 dans le premier intron du gène PPP1R12C du chromosome 19 et invalidation des gènes CD14 et MD2 dans le premier intron du gène CCR5 du chromosome 3 de la lignée cellulaire HEK 293T.

6. Modèle cellulaire selon la revendication 5, **caractérisé en ce que** le nom systématique du modèle cellulaire est le variant 293T/TLR4/CD14/MD2 de cellule rénale embryonnaire humaine (HEK), conservé depuis le 19 mai 2016 au China General Microbiological Culture Collection Center (CGMCC), Institut de Microbiologie, Académie Chinoise des Sciences, N° 1 Route de Beichen Ouest, District de Chaoyang, Beijing, avec le numéro de conservation CGMCC N° 12296.

7. Kit pour détecter des pyrogènes, **caractérisé en ce qu'**il inclut 1) le modèle cellulaire construit par le procédé de construction de l'une quelconque des revendications 1 à 4, ou le modèle cellulaire de la revendication 5 ou 6, 2) un échantillon étalon de pyrogène, et 3) un échantillon témoin d'IL-6 et/ou de TNFα.

8. Trousse pour détecter des pyrogènes selon la revendication 7, **caractérisé en ce que** l'échantillon étalon de pyrogène est une poudre lyophilisée de LPS, l'échantillon témoin d'IL6 est une poudre lyophilisée d'IL6, et l'échantillon témoin de TNFα est une poudre lyophilisée de TNFα.

9. Procédé pour détecter des pyrogènes, **caractérisé en ce qu'**il inclut les étapes suivantes :
1) préparation d'une solution d'échantillon de test et d'une solution d'échantillon étalon de pyrogène ;
2) addition de la solution d'échantillon de test dans un milieu de culture contenant un modèle cellulaire construit par le procédé de construction de l'une quelconque des revendications 1 à 4, ou le modèle cellulaire de la revendication 5 ou 6, et culture à 37 °C pendant 5 heures ;
3) collecte d'un surnageant du produit de culture, et détection des teneurs en IL-6 et/ou TNF-α dans le surnageant.

10. Procédé pour détecter des pyrogènes selon la revendication 9, **caractérisé en ce que** la détection des teneurs en IL-6 et/ou TNF-α dans l'étape 3) est mise en oeuvre par un transfert Western Blot, un ELISA, un test de référence, ou une spectrométrie de masse.
